# EUROPEAN PATENT SPECIFICATION

(11) **EP 0 301 908 B1**
(45) Date of publication and mention of the grant of the patent: **09.02.1994**
(21) Application number: 88307060.9
(22) Date of filing: 29.07.1988
(51) Int. Cl.: C07H 19/073

(54) **1-(3,5-Di-0-benzoyl-2-deoxy-beta-D-threo-pentofuranosyl)thymine and the method of producing the same**
1-(3,5-Di-0-benzoyl-2-deoxy-beta-D-threo-pentofuranosyl)Thymine und Verfahren zu deren Herstellung
Thymine-1-(3,5-di-0-benzoyl-2-déoxy-bêta-D-thréo-pentofuranosyl) et méthode pour sa production

(30) Priority: 29.07.1987 CS 5688/87
(43) Date of publication of application: 01.02.1989
(73) Proprietor: Ceskoslovenska akademie ved, Praha 1 (CS)
(72) Inventor: Hrebanecky, Hubert, Praha 9 (CS); Holy, Antonin, Praha 9 (CS)
(74) Representative: Ablewhite, Alan James

(56) References cited:
- BULLETIN OF THE CHEMICAL SOCIETY OF JAPAN, vol. 52, no. 4, April 1979, pages 1191-1196; J. KIMURA et al.: "Studies on nucleosides and nucleotides. VII. Preparation of pyrimidine nucleoside 5'-phosphates and N3,5'-purine cyclonucleosides by selective activation of the 5'-hydroxyl group"
- CHEMICAL ABSTRACTS, vol. 107, no. 13, 28th September 1987, page 662, abstract no. 115916y, Columbus, Ohio, US; L. KIRIASIS et al.: "Nucleosides. XLIII. Synthesis and properties of O4-alkylthymidines", & NUCLEOTIDES NUCLEOSIDES 1986, 5(5), 517-27

## Description

This invention relates to 1-(3,5-di-O-benzoyl-2-deoxy-β-D-threo-pentofuranosyl)thymine of the formula I and the method of manufacturing the same.

1-(2-Deoxy-β-D-threo-pentofuranosyl)thymine represents a key intermediate in manufacturing 3'-azido-2',3'-dideoxythymidine (AZT, Retrovir®, Zidovudin®) which is used as drug for treatment of AIDS patients. The hitherto existing methods of preparing this compound start from specifically protected 2'-deoxythymidine derivatives and consist in epimerisation of the 3'-hydroxyl group in this compound, e.g. by treatment with methanesulfonyl chloride and then with aqueous alkali (J.P. Horwitz, J. Chua, M. Noel: J. Qrg. Chem. 29, 2076 (1964); J.S. Lin, W. Prusoff: J. Med. Chem. 21, 109 (1978); R.P. Glinski, M. Khan, R. Kalamas, M. Sporn: J. Org. Chem. 38, 4299 (1973). The starting compound, 2'-deoxythymidine, is a natural compound obtained by enzymatic hydrolysis of nucleic acids. Because of low content of DNA in the natural sources and thelr limited availability, the amount of 2'-deoxythymidine prepared from DNA is limited and cannot satisfy the existing great demand. Synthetic approaches to 2'-deoxythymidine are based either on methylation of 2'-deoxyuridine (e.g. A. Holy: Collection Czechoslov. Chem. Commun. 37, 4072 (1972); C.B. Reese, Y.S. Sanghvi: J. Chem. Soc., Chem. Commun. 1983, 877) or on chemical transformation of 5-methyluridine to the 2-deoxyriboside, consisting in the preparation of 2'-chloro-2'-deoxythymidine and its dehalogenation (A. Holy, D. Cech: Collection Csechoslov. Chem. Commun. 39. 3157 (1974)) or in reaction of 2'-O-thiocarbonyl or 2'-O-thiobenzoyl derivatives with tri-n-butyltin hydride (M. J. Robins, J. S. Wilson, F. Hansske: J.Am. Chem. Soc. 105, 4059 (1983); C.H. Kim, V.E. Marquez, S. Broder, H. Mitsuya, J. S. Driscoll: J. Med. Chem. 30, 862 (1987)). The required 5-methyluridine can be prepared by methylation of uridine (K.H. Scheit: Tetrahedron Letters 1965, 1031) or by ribosylation of thymine or its derivatives (M. Prystas, F. Sorm: Collection Csechoslov. Chem. Commun. 31, 1035 (1966); A. Holy, D. Cech: ibid. 39, 3157 (1974)). However, all these methods require expensive starting compounds (2'-deoxyuridine, uridine, D-ribose).

This dependence of producing 3'-azido-2',3'-dideoxythymidine or 1-(2-deoxy-β-D-threo -pentofuranosyl)thymine on 2'-deoxythymidine, as well as other drawbacks of the mentioned procedures, are overcome by the use of 1-(3,5-di-O-benzoyl-2-deoxy-β-D-threo-pentofuranosyl) thymine of the formula I. 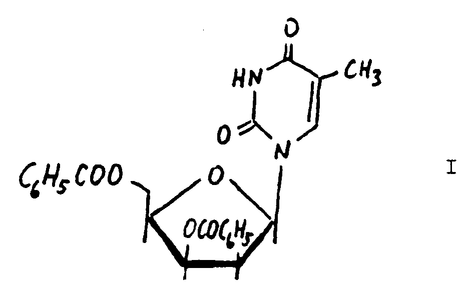
This compound was isolated and characterised in 1979 (see Bull. Chem Soc. of Japan, Vol. 52(4), 1191-1196(1979)). According to the present invention there is provided an improved method for the preparation of 1-(3,5-di-O-benzyl-2-deoxy-β-D-threopentofuranosyl) thymine of the formula I, characterized in that 1,2-O-isopropylidene-α-D-xylofuranose of the formula II 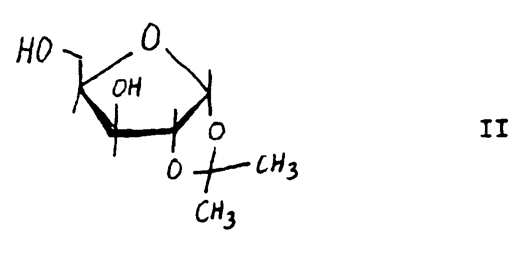
is reacted with 2.5 to 3 molar equivalents of benzoyl chloride in pyridine at 0°C to 20°C and then with 4.5 to 5 equivalents of acetic anhydride in acetic acid in the presence of concentrated sulfuric acid at 0°C, and the obtained intermediate of the formula III 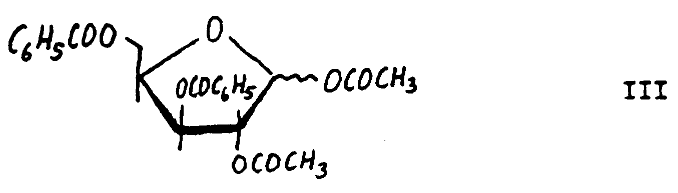
is reacted with 1 to 1.2 molar equivalents of 2,4--bis(trimethylsilyloxy)-5-methylpyrimidine of the formula IV 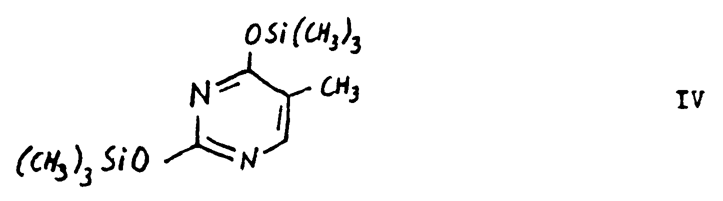
in dichloromethane, 1,2-dichloroethane or acetonitrile in the presence of tin tetrachloride as catalyst, and the resulting crude intermediate of the formula V 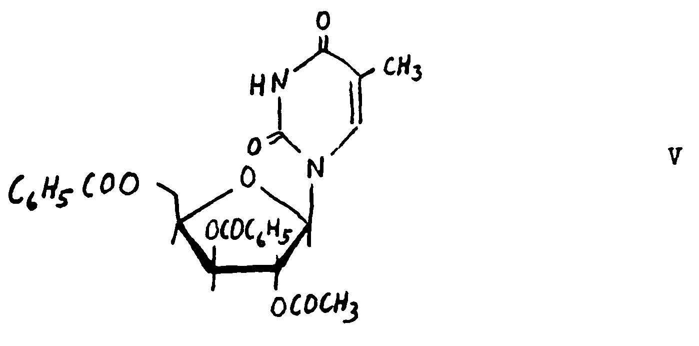
is treated with sulfuric acid (concentration in the reaction mixture 0.1 to 0.2 mol.l⁻¹) in a mixture of water and a water-miscible organic solvent, preferably acetonitrile, at 70°C to 80°C, the resulting 1-(3,5-di-O-benzoyl-β-D-xylofuranosyl)thymine of the formula VI 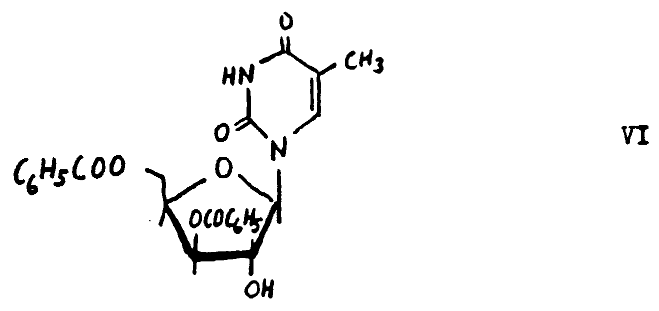
is refluxed with thionyl chloride in acetonitrile, and the obtained crystalline 1-(2-chloro-2-deoxy-3,5-di--O-benzoyl-β-D-threo-pentofuranosyl) thymine of the formula VII 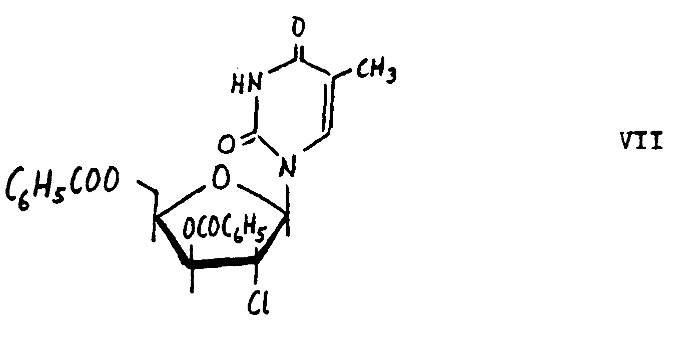
is refluxed with a solution of tri-n-butyltin hydride in benzene or toluene to afford compound of the formula I.

The preparation of the new compound I according to this invention is based on the original method of preparation of the 2′-deoxy-2′-chloro derivative VII, starting from the 1-(β-D-xylofuranosyl)thymine derivative VI, specifically protected with benzoyl groups in positions 3′ and 5′. The remaining free hydroxyl group in compound VI is transformed by simple heating with thionyl chloride in acetonitrile. Although this reaction was already used in transformations of the primary hydroxyl group in position 5′ in 5′-chloro-5′-deoxy derivatives in the ribonucleoside series (Czechoslovak Authors' Certificate No 199 949), its use for the conversion of an isolated secondary hydroxyl group represents a new application of the mentioned principle.

The specifically protected 3′,5′-di-O-benzoyl derivative of the formula VI is prepared in high yield by condensation of a thymine derivative with a suitably protected derivative of 1-O-acetyl-D-xylofuranose that bears in the position 2 an O-acyl group ensuring the stereospecific reaction course using tin tetrachloride as a catalyst for this reaction. The compound of choice is 2,4-bis(trimethylsilyloxy)-5-methylpyrimidine of the formula IV, easily accessible by reaction of thymine with chlorotrimethylsilane in the presence of a tertiary base, or still better, with hexamethyldisilazane, (U. Niedballa, H. Vorbrüggen: J. Org. Chem. 39, 3668 (1974)).

Another important aspect of this invention is the choice of suitable sugar component in the mentioned nucleoside condensation: the required conditions are completely fulfilled by 1,2-di-O-acetyl-3,5-di-O-benzoyl--D-xylofuranose, accessible in high yield from 1,2-O-isopropylidene-α-D-xylofuranose of the formula II, which in turn is prepared by partial hydrolysis of 1,2:3,5-di-O-isopropylidene-D-xylofuranose (e.g. according to A. Holy: Collection Czechoslov. Chem. Commun. 42, 902 (1977)) and which can be distilled in vacuo. The starting diisopropylidene derivative is readily obtainable by reaction of D-xylose with acetone in the presence of a mineral acid (P.A. Levene, A.L. Raymond: J. Biol. Chem. 102, 317 (1933)). The sugar synthon is prepared from the compound of the formula II by benzoylation of the hydroxyl groups in positions 3 and 5 with benzoyl chloride in pyridine (a slight excess of the reagent is sufficient but even a larger excess is tolerated), followed by acetolysis of the 1,2-O-isopropylidene group in a mixture of acetic anhydride and acetic acid in the presence of a mineral acid (preferably sulfuric acid). The reaction sequence is carried out without purification of the intermediates obtained in the individual steps and affords in high yield the protected synthon of the formula III, which in the crude state is sufficiently pure for the nucleoside condensation.

After performing the nucleoside condensation with the compound of the formula IV under the above-described conditions (preferably in 1,2-dichloroethane but also in dichloromethane or acetonitrile) the reaction mixture is decomposed with 5% aqueous solution of sodium hydrogen carbonate and the separated inorganic salts are removed by filtration. The reaction product of the formula V remains in the organic phase and after evaporation and drying is sufficiently pure for the next reaction step which consists in specific acid-catalyzed hydrolysis of the 2'-acetyl group by boiling the compound of the formula V with dilute solution of a mineral acid (e.g. sulfuric acid) in a mixture of water and an organic water-miscible solvent such as acetonitrile, dioxane, tetryhydrofuran, ethanol etc. for several hours. The reaction course can be monitored e.g. by thin-layer chromatography on silica gel. After the end of the reaction the mixture is neutralized and the crude product of the formula VI is taken up in an organic solvent and after drying and evaporation is crystallized or used without further purification in the above-mentioned reaction with thionyl chloride leading to the chloro derivative of the formula VII.

In the last reaction step of preparation of compound I the obtained chloro derivative of the formula VII is reduced. This reaction of the C-Cl bond can be performed by catalytic hydrogenation but this method is not suitable because the thymidine ring might be hydrogenated. The reduction of compound VII can be accomplished using tri-n-butyltin hydride, obtainable by reaction of tri-n-butyltin chloride with lithium aluminium hydride. The reaction is carried out preferably in the presence of a radical catalyst, e.g. azabis(isobutyronitrile), in boiling benzene or toluene. An excess of the reducing agent, though tolerable, is usually not necessary. Upon cooling, the reaction mixture deposits directly the crystalline 1-(3,5-di-O-benzoyl-2-deoxy-β-D-threo-pentofuranosyl)thymine of the formula I in high yield and high purity. This material can be directly used in the preparation of the free nucleoside by methanolysis (in methanol in the presence of sodium methoxide) under usual conditions.

This invention has the following important advantages:
a) New key intermediate in producing an important drug, independent of strategic or expensive materials or compounds of limited accessibility.
b) The method of its producing using domestic materials, first of all cheap D-xylose, available in bulk quantities.
c) The starting compound of the formula II can be prepared using even D-xylose of technical grade because both its 1,2:3,4-di-O-isopropylidene derivative and the compound II can be efficiently purified by distillation in vacuo even on a large scale.
d) Although according to this invention the compound I is prepared in eight steps, all the steps give high yields and the individual intermediates require no purification. The first isolated intermediate which is easily purified by crystallization, is the chloro derivative of the formula VII.
e) The method of preparation does not require extraordinary reaction conditions, special solvents or expensive, inaccessible or sensitive reagents.
f) The compound of the formula I, obtained according to this invention, is very pure and suitable for further use in the preparation of 3′-azido-2′,3′-dideoxythymidine.

The following Examples illustrate the preparation of the compound according to this invention, without limiting in any way the invention.

### Examples of Execution

Example 1. Benzoyl chloride (450 g) is added in the course of 1 hour to an ice-cooled and stirred solution of 1,2-O-isopropylidene-α-D-xylofuranose (244 g; b.p. 150°C/130 Pa) in pyridine (1 litre). The solution is set aside at room temperature for 16 - 20 hours, concentrated at 40°C/2 kPa to 0.5 l, diluted with chloroform (3.5 l) and washed successively with water (0.7 l), 5% sulfuric acid (to acid reaction of the extract), saturated solution of sodium hydrogen carbonate (0.5 l), dried over sodium sulfate, filtered and the solvent is evaporated at 40°C/2 kPa. The residue is dissolved in acetic acid (2.5 l) and acetic anhydride (650 ml), the solution is cooled with ice and conc. sulfuric acid (225 ml) is added in the course of 30 min under stirring. After standing overnight at room temperature, the mixture is poured on ice (20 kg) and extracted with chloroform (3 x 1.5 l). The combined extracts are washed with water (3 x 0.7 l), saturated solution of sodium hydrogen carbonate (until the liberation of carbon dioxide ceases), dried over sodium sulfate, filtered and evaporated at 40°C/2 kPa. Yield 540 g (60%) of compound III. This product (1.22 mol) is dissolved in 1,2-dichloroethane (2 l) and compound IV (330 g; 1.22 mol) is added. Tin tetrachloride (200 ml) is then added under stirring so as the reaction temperature does not exceed 45°C. After standing overnight at room temperature the mixture is diluted with chloroform (3 l) and poured with stirring into 5% aqueous solution of sodium hydrogen carbonate (24 l). The separated precipitate is filtered through a layer of Celite, washed with chloroform (2 x 500 ml) and the combined organic phases of the filtrate are washed successively with 5% solution of sodium hydrogen carbonate (2 l), water (2 l) and saturated solution of sodium chloride (1 l), dried over sodium sulfate, filtered and the solvent is evaporated at 40°C/2 kPa. Yield 605 g of crude compound of the formula V.

A solution of this product in a mixture of acetonitrile (4 l) and 1 mol.l⁻¹ sulfuric acid (600 ml) is heated to 75°C for 16 h, neutralised with solid sodium hydrogen carbonate, filtered and the solid is washed with acetonitrile (250 ml). The combined filtrates are taken down at 40°C/2 kPa to about 600 ml, diluted with ethyl acetate (4 l) and the solution is washed with 5% solution of sodium hydrogen carbonate (200 ml), water (200 ml) and dried over sodium sulfate. After filtration the solvents are evaporated at 40°C/2 kPa to dryness and the thus-obtained compound VI is refluxed with thionyl chloride (233 ml) in acetonitrile (3 l), for 90 min. The mixture is cooled and taken down at 40°C/2 kPa. The residue is dissolved in ethyl acetate (6 l), washed (à 800 ml) with 5% solution of sodium hydrogen carbonate, water and saturated solution of sodium chloride, dried over sodium sulfate, filtered and the solvent is evaporated at 40°C/2 kPa to about 1.5 l. The mixture is cooled and the crystalline product is collected on filter, washed with ethyl acetate, light petroleum and dried. The mother liquors are evaporated at 40°C/2 kPa to drynesss, the residue is crystallized from ethanol and combined with the first crop. Yield of compound VII, m.p. 114 - 115°C, is 310 g (52.5% from the starting compound III).

This product (0.64 mol) is dissolved in toluene (2 l) and 1 mol.l⁻¹ tri-n-butyltin hydride solution in toluene (1.12 l) is added, followed by bis(azoisobutyronitrile) (7.5 g). The mixture is stirred at 85°C for 2 hours, cooled, the crystalline product is collected, washed with toluene (100 ml) and light petroleum and dried. The mother liquors are stripped of solvent at 40°C/2 kPa, the residue is mixed with light petroleum (2 l), the product is filtered, washed with light petroleum and crystallized from ethanol - light petroleum. The total yield of compound I, m.p. 149 - 152°C, is 279 - 280 g.
Example 2. Compound of the formula I (258 g) is stirred with 0.1 mol.l⁻¹ methanolic sodium methoxide (2.75 l). After dissolution (about 4 h) the mixture is set aside at room temperature overnight and then neutralized with a cation-exchanger (H⁺-form), prewashed with methanol. The ion-exchanger is filtered off, washed with methanol (1 litre) and the combined filtrates are taken down at 40°C/2 kPa. The residue is mixed with ether (0.75 l), the crystalline product is collected on filter, washed with ether and dried. The ethereal filtrate is extracted with water (3 x 150 ml), the aqueous extracts are combined, the water is evaporated and the residue is filtered with ether (150 ml), affording a second crop of pure product. Total yield of 1-(2-deoxy-β-D-threo-pentofuranosyl)thymine, m.p. 170 - 172°C, is 132 g (95%).

## Claims

1. A method of producing 1-(3,5-Di-O-benzoyl-2-deoxy-β-D-threo-pentofuranosyl) thymine of the formula I, 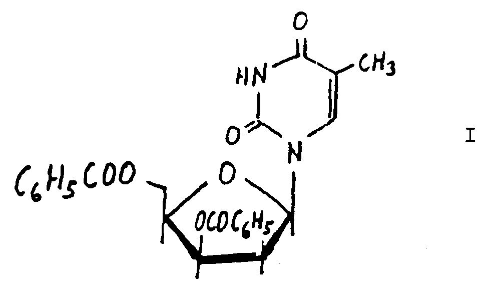 characterised in that 1,2-O-isopropylidene-α-D-xylofuranose of the formula II 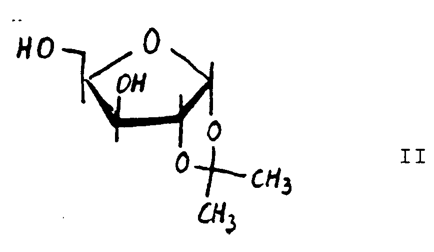 is reacted with 2. 5 to 3 molar equivalents of benzoyl chloride in pyridine at 0°C to 20°C and then with 3. 5 to 5 equivalents of acetic anhydride in acetic acid in the presence of concentrated sulfuric acid at 0°C, and the obtained intermediate of the formula III 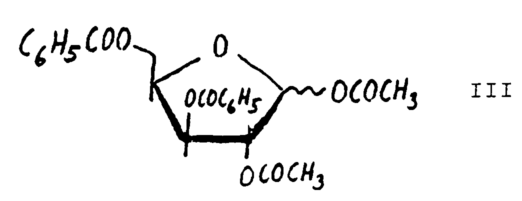 is treated with 1 to 1.2 molar equivalents of 2,2-bis(trimethylsilyloxy)-5-methylpyrimidine of the formula IV 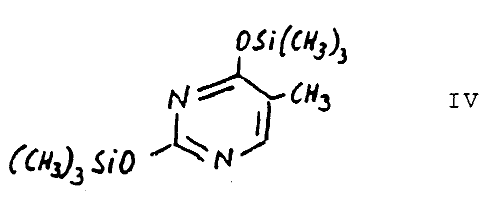 in dichloromethane, 1,2-dichloroethane or acetonitrile as solvent with tin tetrachloride as catalyst, whereupon the obtained intermediate of the formula V 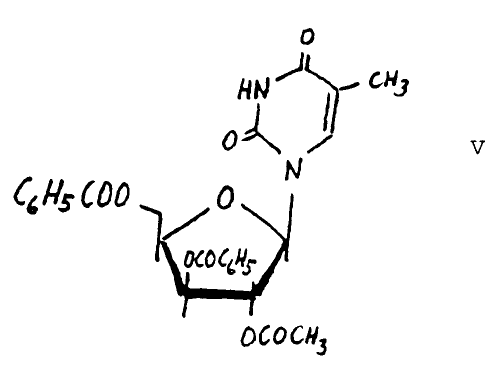 is reacted without purification with sulfuric acid in a mixture of water and a water-miscible organic solvent, preferably acetonitrile, at the total concentration 0.1 to 0.2 mol.ℓ⁻¹ sulfuric acid, at 70-80°C, the obtained 1-(3,5-di-O-benzoyl-β-D-xylofuranosyl)thymine of the formula VI 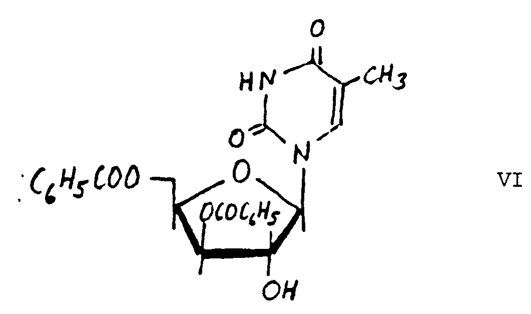 is refluxed with a solution of thionyl chloride in acetonitrile, and the resulting crystalline 1-(2-chloro-2-deoxy-3,5-di-O-benzoyl-β-D-threopentofuranosyl) thymine of the formula VII 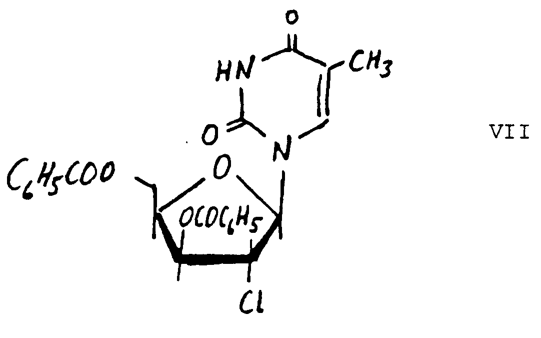 affords the compound of the formula I by refluxing with a solution of tri-n-butyltin hydride in benzene or toluene.

2. The use of the compound of the formula I according in the preparation of 1-(2-deoxy-β-D-threo-pentofuranosyl)thymine.

## Patentansprüche

1. Verfahren zur Herstellung von
1-(3,5-Di-O-benzoyl-2-desoxy-β-D-threo-pentofuranosyl)-thymin mit der Formel I: 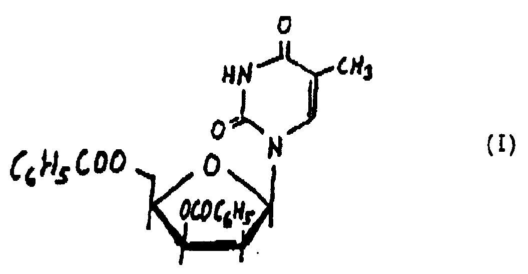 dadurch gekennzeichnet, daß 1,2-O-isopropyliden-α-D-xylofuranose mit der Formel II 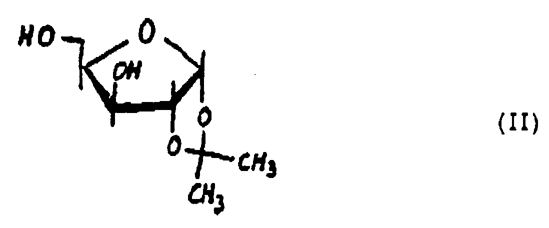 mit 2,5 bis 3 Moläquivalenten Benzoylchlorid in Pyridin bei 0° bis 20°C und danach mit 3,5 bis 5 Äquivalenten Essigsäureanhydrid in Essigsäure in Gegenwart von konzentrierter Schwefelsäure bei 0°C zur Reaktion gebracht und das erhaltene Zwischenprodukt mit der Formel III 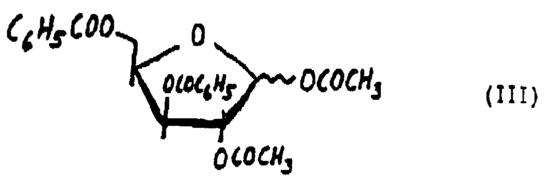 mit 1 bis 1,2 Moläquivalenten 2,2-Bis(trimethylsilyloxy)-5-methylpyrimidin mit der Formel IV 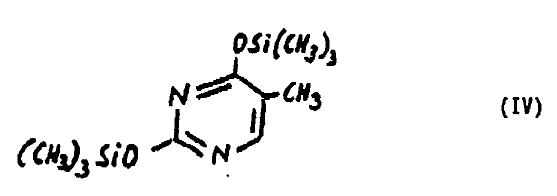 in Dichlormethan, 1,2-Dichlorethan oder Acetonitril als Lösungsmittel mit Zinntetrachlorid als Katalysator behandelt wird, worauf das erhaltene Zwischenprodukt mit der Formel V 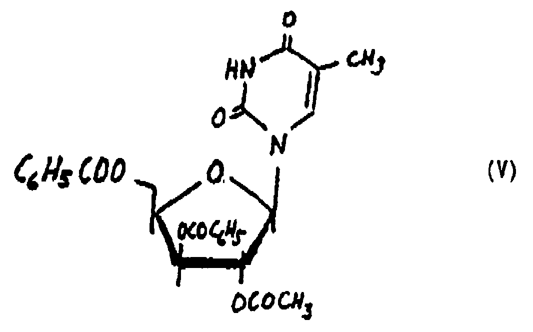 ohne Reinigung in einer Mischung aus Wasser und einem mit Wasser mischbaren organischen Lösungsmittel, vorzugsweise Acetonitril, mit Schwefelsäure in einer Gesamtkonzentration von 0,1 bis 0,2 Mol·l⁻¹ Schwefelsäure bei einer Temperatur von 70-80°C zur Reaktion gebracht wird, das entstandene 1-(3,5-Di-O-benzoyl-β-D-xylofuranosyl)thymin mit der Formel VI 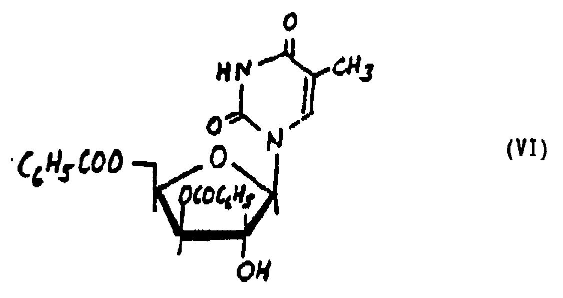 mit einer Lösung von Thionylchlorid in Acetonitril unter Rückflußkühlung erhitzt wird, und das entstandene kristalline 1-(2-Chloro-2-desoxy-3,5-di-O-benzoyl-β-D-threo-pentofuranosyl)thymin mit der Formel VII 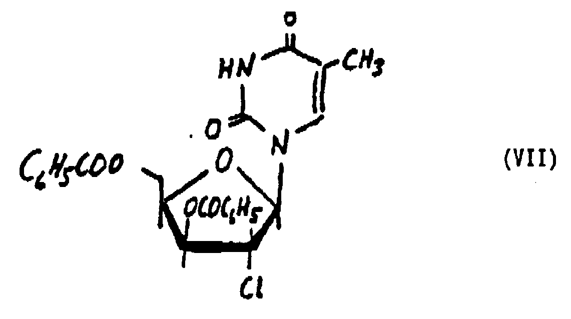 durch Erhitzen unter Rückflußkühlung mit einer Lösung von Tri-n-butylzinnhydrid in Benzol oder Toluol die Verbindung nach Formel I liefert.

2. Verwendung der Verbindung nach Formel I bei der Darstellung von 1-(2-Desoxy-β-D-threo-pentofuranosyl)thymin.

## Revendications

1. Procédé de préparation de la 1-(3,5-di-O-benzoyl-2-désoxy-β-D-thréopentofuranosyl)thymine de formule I 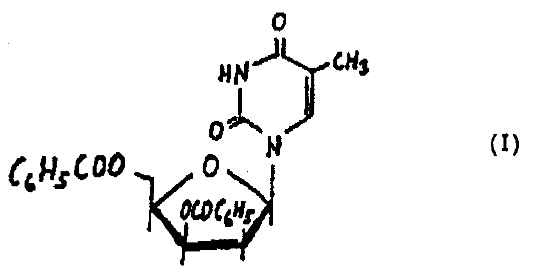 caractérisé en ce qu'on fait réagir du 1,2-O-isopropylidène-α-D-xylofuranose de formule II 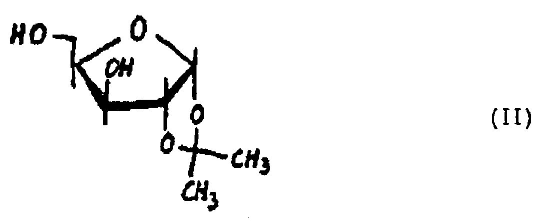 avec 2,5 à 3 équivalents molaires de chlorure de benzoyle dans de la pyridine, à une température de 0°C à 20°C, et ensuite avec 3,5 à 5 équivalents d'anhydride acétique dans de l'acide acétique en présence d'acide sulfurique concentré, à une température de 0°C, et on traite le produit intermédiaire obtenu de formule III 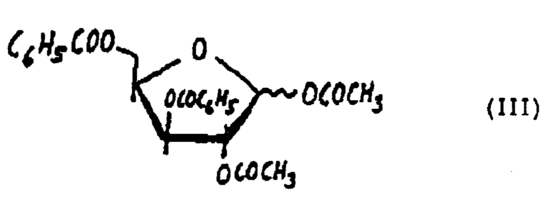 avec 1 à 1,2 équivalent molaire de 2,2-bis(triméthylsilyloxy)-5-méthylpyrimidine de formule IV 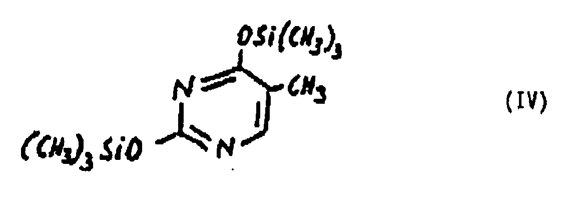 dans du dichlorométhane, du 1,2-dichloréthane ou de l'acétonitrile comme solvant avec du tétrachlorure d'étain comme catalyseur; après quoi, on fait réagir le produit intermédiaire obtenu de formule V 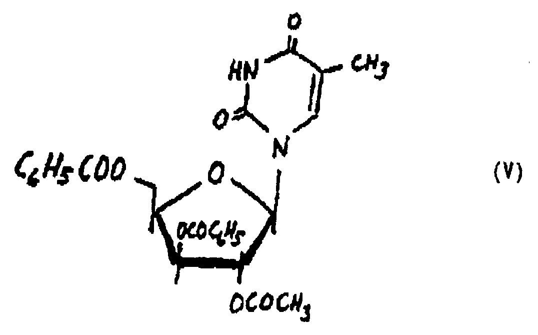 sans purification avec de l'acide sulfurique dans un mélange d'eau et d'un solvant organique miscible à l'eau, de préférence l'acétonitrile, à une concentration totale de 0,1 à 0,2 mole l⁻¹ d'acide sulfurique, à une température de 70-80°C; on chauffe à reflux la 1-(3,5-di-O-benzoyl-β-D-xylofuranosyl)thymine obtenue de formule VI 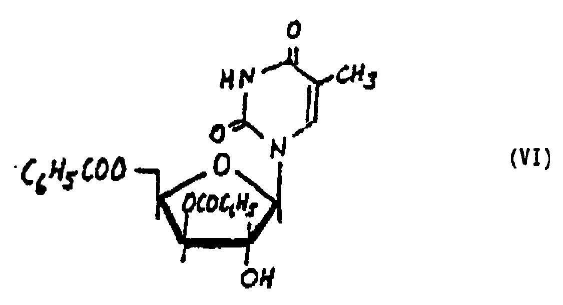 avec une solution de chlorure de thionyle dans de l'acétonitrile, et la 1-(2-chloro-2-désoxy-3,5-di-O-benzoyl-β-D-thréo-pentofuranosyl)thymine cristalline résultante de formule VII 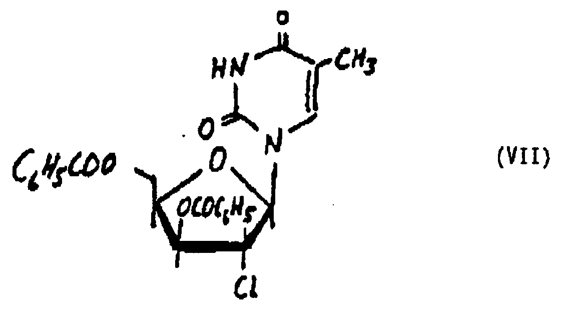 procure le composé de formule I lorsqu'on la chauffe à reflux avec une solution d'hydrure de tri-n-butylétain dans du benzène ou du toluène.

2. Utilisation du composé de formule I conformément à la préparation de la 1-(2-désoxy-β-D-thréo-pentofuranosyl)-thymine.
